# EUROPEAN PATENT APPLICATION

(11) **EP 2 722 058 A1**
(43) Date of publication of application: **23.04.2014**
(21) Application number: 12189206.1
(22) Date of filing: 19.10.2012
(51) Int. Cl.: A61K 49/00, A61K 9/16, A61K 9/20, A61K 9/00, A61K 9/28

(54) **Solid oral composition containing dyes for use in endoscopic diagnosis**

(71) Applicant: Cosmo Technologies Ltd, Dublin 1 (IE)
(72) Inventor: Moro, Luigi, I-21050 Cairate (VA) (IT); Repici, Alessandro, I-10123 Torino (IT)
(74) Representative: Bartorelli, Luisa

(57) **Abstract**

Herein described are solid oral compositions of dyes for use in diagnostic endoscopy, preferably colon endoscopy.

## Description

Endoscopy is an exceptionally important diagnostic technique for the diagnosis of inflammatory, ulcerative, and neoplastic pathologies of the gastrointestinal tract. Actually, endoscopy allows observing - from inside the lumen - the state of preservation and development of the mucosa that covers the gastrointestinal cavity, as well as the surface spraying thereof, the presence of deformation, and/or neoformations, and/or ulcerations.

Increasingly more powerful and sophisticated endoscope probes have considerably improved this technique. The progress of the materials employed has also improved performance in terms of illumination technologies and resolution power.

More recently, there has been an improvement of the conventional diagnostic-therapeutic aspects involving image magnification and vital dyes, used to locally develop a contrasting colour capable of amplifying the resolution diagnostic power of the conventional technique. The use of dyes in diagnostic endoscopic procedures is described by "chromoendoscopy", particularly useful for identifying suspicious areas displaying degenerative characteristics.

The use of colouring is generally adopted in the second part of the endoscopic analysis, during the step of withdrawing the endoscopic probe, and after accurately cleaning the mucosa tract to be examined. Currently, the dye is applied to the mucosa by spraying a certain volume of a dye-containing solution using a catheter or capillary pipe directly inserted into the working channel of the endoscopic probe.

The diffusion of the dye on the cell surface or the extent of absorption by the vital cells markedly differentiates the cells with normal vitality from those cells, such as neoplastice cells, in the advanced replication stage.

The dyes usually used are mainly, but not exclusively, the following: methylene blue, congo red, carmine indigo, and/or toluidine blue.

Methylene blue and toluidine blue are uniformly absorbed by the whole intestinal mucosa but that absorption is reduced in an inflammatory environment, particularly as the phlogosis, i.e, inflammation, worsens. Due to this characteristic, the two dyes are also useful to ascertain whether inflammatory processes are in remission, and are also useful in distinguishing between pseudopolyps and true polyps. Indeed, inflamed or malignant/premalignant colonic epithelium exhibits decreased cytoplasm and goblet cells that are either reduced in amount or absent. These alterations result in decreased uptake of methylene blue and endoscopic appearance of focal light blue or pink (unstained) or heterogeneously stained (specked) mucosa in contrast to a more uniform staining pattern when colonic mucosa is not affected by pathologic processes. Differently from this concept, carmine indigo is not absorbed by cells and functions as a contrast agent increasing visibility of mucosal structures and enhancing details of normal and abnormal colonic patterns. Carmine indigo thus finds application in long duration inflammatory forms and can be used to highlight flat lesions, which can contain tumoral forms, which are difficult to detect with conventional white light endoscopy that does not employ contrasting colours.

Within the dyeing procedure, it should be observed that use thereof reveals several practical problems that can be difficult to resolve due to the challenges involved in applying the dye. First and foremost the pharmacy of the institute where the endoscopy is performed should be capable of preparing solutions with concentrations of dye generally ranging from 0.1% to 1%; then the dye should be dispensed (using a dedicated spray catheter) uniformly so as to cover homogeneously the mucosal surface subject of the evaluation.

Furthermore, the sprayed dye excess is to be removed after a few minutes through washing and sucking operations. That removal of excess dye requires additional time after each repetition of the dyeing spray process during the colonoscopy. The process, consequently, is time consuming for both nurses and physicians and makes it difficult to maximize the efficiency of the schedule of endoscopic procedures. The procedure is sufficiently rare that it tends to be operator-dependent, requiring a dedicated learning curve to obtain the right level of expertise to be able to evaluate the specific staining patterns obtained and their significance.

The need for the simultaneous presence of these precise conditions contributes to the difficulty of executing the chromoendoscopy procedure. Those difficulties have resulted in the procedure being carried out by only a minority of endoscopy units in hospitals and nursing homes specialized in gastroenterology.

Furthermore, other problems have resulted. The conventional local spraying of a solution on the mucosal wall may fail to reveal forms that are latent but still too small to detect and may fail to reveal the degenerative processes of the digestive system. Moreover, locally spraying a solution can result in a short performance time of the dye. In particular, the time between spraying of the dye and observation is generally only a few seconds or a couple of minutes, a period known to be too short for allowing a consistent absorption of the dye to provide good contrast development and also achievement of good staining efficacy. Those issues may make it difficult for the endoscopist to intervene to obtain good detection and evaluation, as for example, in a biopsy.

Furthermore, the experience of each endoscopist who performs the procedure is somewhat subjective, additionally generating problems in the execution of both the endoscopic and related diagnostic evaluations. As a practical difficulty, such subjectivity resulting from the experience and convenience of the operator can undesirably lead to great variability in results. And the experience of the endoscopist plays an important role: the more experienced endoscopist, compared to the less experienced endoscopist, may spot suspicious areas when the dye is sprayed according to the current chromoendoscopy, further exacerbating the subjectivity of the test results. Significant variability in test results can also result from the apparatus used, as well as from the acceptability of a particular patient to the diagnostic evaluation practice. Thus, there arises the need of providing further improvement in both simplicity and safety from use of a dye in diagnostic endoscopies. It is desirable to improve the means of administration to provide a homogeneous and complete distribution of the dye for an improved effect in evaluating a treated area.

And as will be evident from above, it is desirable to obtain improvements that will increase the objectivity of the endoscopic evaluation to allow an improved diagnostic evaluation.

Particularly, in the case of colonic endoscopy (colonoscopy), a need still exists for providing an improved mucosal staining and ameliorating the efficacy of the diagnostic endoscopy evaluation.

It has been surprisingly discovered that a specific solid composition containing at least one dye and at least one physiologically acceptable excipient, orally administered according to a defined fractionated schedule prior to endoscopy, can provide an improved mucosal staining. And, increasing the objectivity of the endoscopy, the solid composition disclosed herein can also provide an improved detection characterization in endoscopic diagnosis.

Thus, disclosed herein is a solid composition containing at least one dye in association with at least one physiologically acceptable excipients which comprises:
a) a matrix which comprises lipophilic compounds with melting point below 90°C, and optionally amphiphilic compounds, in which matrix at least one dye is at least partly incorporated,
b) an outer matrix which comprises hydrophilic compounds, in which the lipophilic matrix, and optionally the amphiphilic matrix are dispersed;
a) optionally other physiologically acceptable excipients;
b) optionally a gastro-resistant coating
for use in endoscopic diagnosis characterised in that two or more unit dosages of the solid composition are orally administered to a human according to a fractionated schedule in which a total amount from 100 to 400 mg of said at least one dye is administered to a human in the 48 hours prior to endoscopic diagnosis. For example, said at least one dye is administered to a human in the 24 hours prior to endoscopic diagnosis.

In the alternative, the matrix consists of lipophilic compounds with melting point below 90°C, and optionally amphiphilic compounds, in which matrix at least one dye is at least partly incorporated, and the outer matrix consists of hydrophilic compounds, in which the lipophilic matrix, and optionally the amphiphilic matrix are dispersed.

Said two or more unit dosages are, for example, four, six or eight unit dosages administered in the 48 hours prior to endoscopy, such as in the 24 hours prior to endoscopy.

Useful dyes according to the present disclosure can be, for example, selected from among congo red, carmine indigo, methylene blue, toluidine blue or mixtures thereof; for example, the dye is methylene blue.

According to the disclosure herein, methylene blue can be in anhydrous or hydrated forms, such as the trihydrate form.

However, according to the disclosure other biocompatible dye substances can also be used, as long as they are provided with a toxicity profile that does not represent an obstacle to oral systemic administration thereof.

A "fractionated schedule" according to the disclosure means that the total amount of the dye to be orally administered before colonoscopy is divided in two or more unit dosages to obtain a pre-defined administration schedule. The dose fractionation can reduce the possibility that staining will be lost due to unwanted strange intestinal motility. And the dose fractionation can facilitate the spreading of the blue staining matrices.

The endoscopic diagnosis as disclosed herein is directed to the gastro-intestinal tract, such as the colon (colon endoscopy or colonoscopy). According to the anatomical classification, the colon is divided into four (4) regions of interest (ROI), namely (1) ascending colon (AC), (2) transverse colon (TC), (3) descending colon (DC), and (4) rectosigmoid (RES).

As disclosed herein, the total dose amount of said at least one dye is, for example, from 50 to 500 mg, such as from 100 to 400 mg, such as from 100 to 250 mg, and further such as 200 mg.

As disclosed herein, the unit dosage of the composition contains, for example, from 20 to 200 mg by weight of the at least one dye. For example, said unit dosage contains about 25 mg or about 50 mg, such as 25 mg or 50 mg, by weight of said at least one dye.

According to an embodiment disclosed herein, eight unit dosages of the composition, each containing about 25 mg, such as 25 mg, by weight of said at least one dye, are administered to said human in the 48 hour period prior to endoscopic diagnosis. According to another embodiment disclosed herein, six unit dosages of the composition, each containing about 25 mg, such as 25 mg, by weight of said at least one dye, are administered to said human in the 48 hour period prior to endoscopic diagnosis. According to a yet one other embodiment disclosed herein, four unit dosages of the composition of the invention, each containing about 25 mg, such as 25 mg, by weight of said at least one dye, are administered to said human in the 48 hour period prior to endoscopic diagnosis.

According to a further embodiment disclosed herein, four unit dosages of the composition, each containing about 50 mg, such as 50 mg, by weight of said at least one dye, are administered to said human in the 48 hour period prior to endoscopic diagnosis. According to a yet further embodiment disclosed herein, two unit dosages of the composition disclosed herein, each containing about 200 mg, such as 200 mg, by weight of said at least one dye, are administered to said human in the 48 hour period prior to endoscopic diagnosis.

As disclosed herein, to facilitate the mucosal observation through the endoscope by the endoscopist, said human, prior to endoscopic diagnosis, can be subjected to a bowel cleansing preparation by the administration of bowel cleansing solution to quantitatively remove the stool and mucous residuals. This cleansing operation is carried out generally in the 48 hour period prior to endoscopic diagnosis, such as in the 24 hour period prior to endoscopic diagnosis or, as found to be practical for carrying out a colonoscopy in the late afternoon, also in the same day.

The colon cleansing preparation could be administered by drinking the volume fractions of the cleansing solution consecutively during the day before or, with the so called "split" version, by dividing the administration of the cleansing solution volume in two parts, one to be administered the day before the colonoscopy and one to be administered in the morning of the day in which the colonoscopy is to be subsequently performed. The bowel cleansing solution is used for cleaning and washing the intestinal tract and mucosa before the endoscopic diagnosis. The bowel cleansing solution is, for example, a saline and/or polyethylenglycol (PEG) aqueous solution, such as a polyethylene glycol aqueous solution. As a further example, said aqueous solution contains, excluding water, from 50%.to 95% by weight of polyethylene glycol, sometimes also including into that solution, salts and flavours, such as sodium salts, potassium salts, ascorbic acid, and mixtures thereof. For example, sodium sulphate, sodium sulphate anhydrous, sodium chloride, sodium ascorbate, sodium bicarbonate, sodium salt of ascorbic acid, potassium sulphate, potassium chloride and mixtures thereof can be used. As a further example, the bowel cleansing solution is an aqueous solution of commercially available products sold under such names as Moviprep^{®} or Golytely^{®}, Nulytely^{®}, or Halflytely^{®}, or Movicol^{®}, or Macro-P^{®}, or Colirei^{®}, or Isocolan^{®} or Selg 1000^{®}.

However, as disclosed herein, also other bowel cleansing solutions or preparations can be used, as long as they are provided with a toxicity profile that does not represent an obstacle to oral systemic administration thereof. For example, bowel cleansing solution containing only salts or other small chemical laxatives, but not PEG, are available on the market under the brands Phospho-Lax^{®} or Picoprep^{®} or Suprep^{®}. Also different bowel preparation procedures can be used.

As disclosed herein, the cleansing solution can be administered in a total amount of four litres, which can be fractionated in one or more unit dosages, for example, in four unit dosages of about one litre each.

The solid composition, as disclosed herein, can be thus administered together and/or after the intake of each unit dosage of said bowel cleansing solution, prior to the endoscopic diagnosis. Afterwards, still water can also be additionally administered, if necessary.

As disclosed herein" four unit dosages of the composition, each containing about 25 mg, such as 25 mg, by weight of said at least one dye, are orally administered to a human according to a fractionated schedule in which a total amount of about 100 mg, such as 100 mg, of said at least one dye is administered to said human in the 48 hour period prior to the endoscopic diagnosis in:
- 1 solid oral composition after intake of the 1^{st} litre of bowel cleansing solution;
- 1 solid oral composition after intake of the 2^{nd} litre of bowel cleansing solution;
- 1 solid oral composition after intake of the 3^{nd} litre of bowel cleansing solution; and
- 1 solid oral composition after intake of the 4^{nd} (and last) litre of bowel cleansing solution.

As disclosed herein, eight unit dosages of the composition, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 2 solid oral compositions after intake of the 1^{st} litre of bowel cleansing solution;
- 2 solid oral compositions after intake of the 2^{nd} litre of bowel cleansing solution;
- 2 solid oral compositions after intake of the 3^{nd} litre of bowel cleansing solution; and
- 2 solid oral compositions after intake of the 4^{nd} (and last) litre of bowel cleansing solution.

For example, eight unit dosages of the composition as disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 0 solid oral compositions after intake of the 1^{st} litre of bowel cleansing solution;
- 2 solid oral compositions after intake of the 2^{nd} litre of bowel cleansing solution
- 3 solid oral compositions after intake of the 3^{nd} litre of bowel cleansing solution; and
- 3 solid oral compositions after intake of the 4^{nd} (and last) litre of bowel cleansing solution.

As a further example, eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 0 solid oral compositions after intake of the 1^{st} litre of bowel cleansing solution;
- 4 solid oral compositions after intake of the 2^{nd} litre of bowel cleansing solution;
- 4 solid oral compositions after intake of the 3^{nd} litre of bowel cleansing solution; and
- 0 solid oral compositions after intake of the 4^{th} litre of bowel cleansing solution.

As a yet further example, eight unit dosages of the composition as disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 48 hour period prior to the endoscopic diagnosis in:
- 0 solid oral compositions after intake of the 1^{st} litre of bowel cleansing solution;
- 3 solid oral compositions after intake of the 2^{nd} litre of bowel cleansing solution;
- 3 solid oral compositions after intake of the 3^{nd} litre of bowel cleansing solution; and
- 2 solid oral compositions after intake of the 4^{th} litre of bowel cleansing solution.

As further disclosed within, four unit dosages of the composition as disclosed herein, each containing about 25 mg, such as 25 mg, by weight of said at least one dye, are orally administered to a human according to a fractionated schedule in which a total amount of about 100 mg, such as 100 mg, of said at least one dye is administered to said human in the 48 hour period prior to the endoscopic diagnosis in:
- 0 solid oral composition after intake of the 1^{st} litre of bowel cleansing solution;
- 1 solid oral composition after intake of the 2^{nd} litre of bowel cleansing solution;
- 1 solid oral compositions after intake of the 3^{nd} litre of bowel cleansing solution; and
- 2 solid oral compositions after intake of the 4^{nd} (and last) litre of bowel cleansing solution.

As further disclosed within, two unit dosages of the composition as disclosed herein, each containing about 200 mg, such as 200 mg, by weight of said at least one dye, are orally administered to a human according to a fractionated schedule in which a total amount of about 400 mg, such as 400 mg, of said at least one dye is administered to said human in the 48 hour period prior to the endoscopic diagnosis in:
- 0 solid oral composition after intake of the 1^{st} litre of bowel cleansing solution;
- 1 solid oral composition after intake of the 2^{nd} litre of bowel cleansing solution;
- 1 solid oral compositions after intake of the 3^{nd} litre of bowel cleansing solution; and
- 0 solid oral compositions after intake of the 4^{nd} (and last) litre of bowel cleansing solution.

As even further disclosed herein, six unit dosages of the composition, each containing about 25 mg, such as 25 mg, by weight of said at least one dye, are orally administered to a human according to a fractionated schedule in which a total amount of about 150 mg, such as 150 mg, of said at least one dye is administered to said human in the 48 hour period prior to the endoscopic diagnosis in:
- 2 solid oral composition at the beginning of bowel preparation, before intake of the 1^{st} litre of bowel cleansing solution;
- 2 solid oral compositions after intake of the 1^{st} litre of bowel cleansing solution;
- 2 solid oral compositions after intake of the 2^{nd} litre of bowel cleansing solution
- 0 solid oral compositions after intake of the 3^{nd} litre of bowel cleansing solution; and
- 0 solid oral compositions after intake of the 4^{nd} (and last) litre of bowel cleansing solution.

As yet another further example, the above indicated administration schedule can be carried out applying also the "split" bowel cleansing procedure. In such a case, the tablet administration is split over the two days of bowel cleansing preparation, maintaining the relevant schedule here described. Examples of the split preparation, according to further example disclosed herein, are here below detailed:
eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 24 hour period prior to the endoscopic diagnosis in a split preparation procedure, where:
   - 3 solid oral compositions after intake of the 1^{st} litre of bowel cleansing solution the day before colonoscopy;
   - 3 solid oral compositions after intake of the 2^{nd} litre of bowel cleansing solution the day before colonoscopy;
   - 2 solid oral compositions after intake of the 3^{nd} litre of bowel cleansing solution the same day of colonoscopy; and
   - 0 solid oral compositions after intake of the 4^{th} litre of bowel cleansing solution the same day of colonoscopy.

Alternatively, as a further example, eight unit dosages of the composition disclosed herein, each containing about 25 mg, such as 25 mg, by weight of said at least one dye, are orally administered to a human according to a fractionated schedule in which a total amount of about 200 mg, such as 200 mg, of said at least one dye is administered to said human in the 24 hour period prior to the endoscopic diagnosis in a split preparation procedure, where:
- 0 solid oral compositions after intake of the 1^{st} litre of bowel cleansing solution the day before colonoscopy;
- 6 solid oral compositions during the intake of the 2^{nd} litre of bowel cleansing solution the day before colonoscopy;
- 2 solid oral compositions after intake of the 3^{nd} litre of bowel cleansing solution the same day of colonoscopy; and
- 0 solid oral compositions after intake of the 4^{th} litre of bowel cleansing solution the same day of colonoscopy.

The solid composition disclosed herein can be a controlled release composition. The expression "controlled release" of the composition disclosed herein is used to indicate a composition capable of releasing the dye in a selective site-time manner, i.e. progressive in the areas of interest. Thus, such expression comprises the "prolonged, sustained, extended, delayed or modified" release definition.

The technology suitable for the formulation of controlled release composition disclosed herein can be selected from the colonic specific release technologies, utilized with matrix structures, and the reservoir structure as systems, using dissolution controlling mechanisms and technologies known in the art, such as diffusion, swelling, and macromolecular relaxation.

The oral composition disclosed herein can be formulated according to the multimatrix technology commercially known under the trade mark MMX^{®}, described in the international patent applications WO2011/107945, WO00/76481 and WO00/76478 and copending U.S. patent application Serial No. 13/602,875 filed on 4 September 2012, the disclosures of which relevant to multimatrix technology are specifically incorporated by reference herein.

Suitable lipophilic compounds as disclosed herein can be selected from saturated, unsaturated and hydrogenated long chain alcohols, saturated and unsaturated and hydrogenated fatty acids, salts thereof, esters and amides, mono-, di- and triglycerides of fatty acids, polyethoxylated derivatives thereof, waxes, ceramides, cholesterol, cholesterol derivatives and mixtures thereof having a melting point lower than 90°C, such as from 40 to 90°C, and further such as from 60 to 70°C.

Suitable amphiphilic compounds as disclosed herein can be selected from among polar lipids of type I and II (lecithin, phosphatidylcholine, phosphatidylethanolamine, and mixtures thereof), ceramides, glycol alkyl ethers (such as for example, diethylene glycol monomethyl ether), alkyl sulfate and sulfosuccinate salts, and mixtures thereof.

Suitable hydrophilic compounds as disclosed herein can be chosen from compounds forming hydrogel (i.e. compounds which form hydrogel on contact with aqueous solvents), such as those selected from among polymers and copolymers of acrylic acid, copolymers of methacrylic acid, alkyl vinylpolymers, alkyl celluloses, hydroxyalkyl celluloses, carboxyalkyl cellulose, modified and/or plurisubstituted celluloses, polysaccharides, dextrins, pectins, starches, complex starches and starch derivatives, alginic acid, synthetic rubber, natural rubber, polyalcohols and mixtures thereof. Hydrogens are compounds which when passing from the dry state to the hydrated one undergo so-called "molecular relaxation", namely a remarkable increase in mass and weight following the coordination of a large number of water molecules by the polar end groups present in the polymeric chains of the excipients themselves.

A suitable gastro-resistant coating, as disclosed herein, can be chosen from polymers of acrylic acid, polymers of methacrylic acid, copolymers of acrylic acid, copolymers of methacrylic acid, cellulose derivatives (such as for example cellulose acetate phthalate) hydroxybutyrate-based polymers, shellac and mixtures thereof. Such gastro-resistant coatings of the invention can also be combined with plasticisers, opacifiers, dyes and mixtures thereof.

The administration of a controlled release composition as disclosed herein actually allows releasing the dye contained in the composition precisely starting from the gastrointestinal segment intended to be subjected to endoscopic evaluation, such as in the intestinal regions and even further such as in the colonic regions.

The composition as disclosed herein is formulated in forms chosen from tablets, capsules, granules, microgranules, and pellets, such as in the form of a coated tablet, further such as in the form of gastro-protected tablets.

The capsule form disclosed herein may in turn contain granules, microgranules and/or pellets.

For example, the composition descirbed herein may be formulated in the form of gastro-resistant tablets or in the form of a capsule containing gastro-resistant granules, gastro-resistant microgranules and/or gastro-resistant pellets.

Furthermore, the composition disclosed herein may be formulated in a double layer form, such as a double layer tablet.

As disclosed herein, in case of colonoscopy, two or more unit dosages of the compositions disclosed herein may be provided for the oral administration of two or more unit dosages of the compositions described herein, such as a controlled release tablet, so as to prevent the dye from being dispersed into areas of the digestive tract not intended to be subjected to colonoscopy, such as, for example, the stomach, duodenum and jejunum.

For the preparation of controlled release compositions, one or more dyes can be formulated alongside substances capable of imparting progressive or massive or controlled or prolonged dissolution properties to the formulation. In addition, the formulation is coated with substances capable of dissolving solely upon reaching a specific pH, generally running from pH 5 to pH 7, that pH being typical of the section intended to be subject to the intestinal endoscopic evaluation.

Upon reaching the intestinal section of interest, characterised by a specific pH value at which the gastro-protective coating starts dissolving, the dissolution of the dye can be controlled in terms of speed so as to ensure that it occurs within the time required by the intestinal transit, such as the time to reach the colon, generally running from 4 to 24 hours.

As disclosed herein, the dye/s is/are first mixed or granulated with the material capable of forming a lipophilic matrix, such as in the presence of one or more amphiphilic substances with surfactant properties, and lastly this matrix of powders, at any degree of aggregation, is inserted into a dominant structure formed by polymers or copolymers of the hydrophilic type, also known as hydrogels, in the anhydrous state or with some residual moisture value.

Alternatively, still according to a typical application of this technology, the dye/s should be first mixed or granulated with the material capable of forming a lipophilic matrix, and after granulation this matrix structure, at any degree of aggregation, is inserted into a dominant structure formed by polymers or copolymers of hydrophilic type in anhydrous state or with some residual moisture value in the presence, for example, of one or more amphiphilic substances with surfactant properties. Subsequently the final mixture is subjected to compression.

A gastro-protective coating film, capable of preventing the dissolution of the composition in a strongly acid environment, can be lastly applied to the surface of the compositions.

Upon swallowing, such a multimatrix coated composition can be protected from contact with gastric and intestinal acids up to reaching an environment with suitable pH, such as greater than 5 or 7, where the gastro-protective coating is solubilised and where the dissolution program - which will lead it to progressively distribute the dye inserted in the formulation simultmeously with the progress of transit within the digestive cavity - starts.

The endoscopic diagnosis disclosed herein is aimed at the diagnosis of inflammatory, ulcerative, pre-neoplastic, dysplastic and/or neoplastic pathologies and/or alterations of the gastrointestinal tract, such as of the colon and further such as the right part of the colon.

For example, the endoscopic diagnostic evaluation disclosed herein can be aimed at the diagnosis of cancerous forms, precancerous forms, interval cancers, adenomas, carcinomas, serrated lesions, dysplasias, polyps, pseudopolyps, pre-polyps hyperplastic lesions and different inflammatory pathologies and/or lesions of the gastrointestinal tract, such as of the colon and further such as of the right part of the colon.

The endoscopic diagnosis of the right part of the colon can also be aimed at the diagnosis of right colon adenomas, right colon polyps, serrated adenomas and right serrated lesions or interval cancers.

An interval cancer relates to lesions able to become cancers (tumours) in the time between two consecutive colon endoscopies (colonoscopies). Such time generally corresponds to a period of 2-5 years.

The oral composition disclosed herein can be aimed to increase and to improve the diagnosis of those small size lesions and flat lesions that are mostly missed during white light colonoscopy. As used herein, the term "small size" is a size equal to or less than 10 nm, such as equal or less than 5 mm. For example, , polyps, adenomas and serrated lesion of the right colon of size less than 5 mm in diameter are considered to be "small size."

The size is determined as the diameter of lesion estimated or measured by using a standard foreign body forceps.

These right colon lesions are in fact considered difficult to be seen and detected in this field, because of the anatomical conformation of the colon mucosal tissues and the possibility to have an unclean mucosal surface, that would make the lesion's detection very difficult in standard white light colonoscopy practice.

Also, the smaller colon lesions are the more difficult to be selected because of the possibility to be confused with the colonic plicas, as well as the possibility of having an unclean mucosal surface that hides such smaller lesions, thus making those smaller lesions difficult to detect.

As disclosed herein, the endoscopic diagnosis can also be aimed at the diagnosis of the above mentioned pathologies and/or lesions in a human previously suffering from at least another inflammatory pathology as, for example, Inflammatory Bowel Disease (IBD), Ulcerative Colitis or Crohn's Disease.

In that case, said human is indicated to be a "more risky patient". In this kind of patients, in fact, the risk of subsequent pathologies and/or lesions of the intestinal and colonic mucosa is much higher than normal because the mucosa is affected by chronic flogistic processes that in the long-term may be associated with uncontrolled cell proliferation and neoplastic development. Particularly, the risk significantly increases at colonic level where for example colon carcinoma and/or colon dysplasia and/or intraepithelial neoplasias can more likely arise in patients with long-standing ulcerative colitis and Crohn's disease.

A first advantage of the oral composition disclosed herein is to provide an improved staining quality and staining efficacy in the area to be investigated by the endoscopic diagnostic evaluation, such as the colon regions (ascending, descending, rectosigmoid and transverse colon) and even further such as the right part of the colon.

This improved staining quality is related to a number of different factors. First, the dye is quite homogenously delivered throughout the entire length of the bowel according to the multi-matrix delivery system and the specific schedule of dye administration which ensures long-lasting and anatomically consistent availability of the coloring substance. Second and foremost, the disclosure herein allows for the first time a certain interval time between the dye contact with the colonic mucosa and the endoscopic procedure. This interval time is relevant, allowing for proper dye absorption in the mucosa which becomes consistently coloured thanks to the incorporation of the blue substance into the cells. Selective dye absorption is considered the pivotal mechanism of action of vital dyes like methylene blue.

Indeed this absorption and the consequent enhanced contrast is minimally obtained when the dye is sprayed during the endoscopic procedures. The absorption is maximized when a certain interval lasts between dye delivery and endoscopic procedure.

The third factor leading to an improved staining is strictly related to colonic anatomy. Indeed the right colon has a larger lumen and a greater mucosal surface as compared to other colonic segments.

According to these facts and because of a gravity issue (during the endoscopic procedure the patients lay down in a supine position) when the dye is sprayed at the time of endoscopic procedure, the dye tends to distribute in a patchy mode, for example, in the most downward part of the mucosa (because of gravity).

Differently from this situation, in the condition of a targeted oral delivery of the dye with an MMX mechanism, at least 5 hours before the procedure, the availability of a significant dosage of the dye and the presence of abundant aqueous material (the bowel prep solution), taken together with peristaltic movements of the right colon, optimize the diffusion of the dye and contact of the dye with the different mucosa segments of the right colon.

Once the colonic mucosa is consistently and persistently coloured with methylene blue, the resulting diagnostic advantage is an increased ability to detect mucosal abnormalities according to different actions specifically related to the dye. First and foremost, areas of mucosa with inflammatory or neoplastic changes tend to decrease the uptake of the dye thus resulting in unstained areas which are easily distinguished (during the endoscopic procedures) from normal mucosa which exhibits a homogeneous staining pattern.

Another advantage of the oral composition disclosed herein is to provide an improved detection of the pathological and/or not pathological lesions in the area to be investigated by the endoscopic diagnosis, such as the colon regions in all its anatomical segments (ascending, descending, rectosigmoid and transverse colon). For example, the right part of the colon can be the more accurately stained area.

The oral composition disclosed herein allows, thanks to a different uptake of the dye in the intercellular and intracellular spaces, a contrast enhancing efficacy of the dye in perceiving the deep mucosal tissue structure with the cripta and the gland ducts, thus improving the exact definition of the lesions and/or the borders of the lesions that the endoscopist has to identify and take out. An improved definition of the mucosal tissue structure and organization of the lesions is ensured, allowing for early detection of the lesions.

The better definition of the lesions provided by the oral composition and administration schedule disclosed herein facilitates increased specificity and sensitivity of the detection of the lesions, thus reducing the occurrence of false-negatives and false-positives and allowing pathological or malignant areas to be more correctly identified and detected. In other words, the specific oral solid composition disclosed herein and the administration schedule of the solid composition defined herein provide the improved contrast of the dye on the mucosa tissue structures.

In particular, the oral solid composition and administration schedule disclosed herein enable very early detection of colon dysplasias and colon carcinomas, particularly of those resutlting from previous ulcerative colitis or Crohn's disease.

A further advantage of the oral solid composition and administration schedule disclosed herein is to provide a maximized local bioavailability of the dye and an optimized biological effect of the same.

In fact, it should be noted that the dye in accordance with the disclosure herein is allowed to be locally released with an homogeneous spreading exactly in the place subjected to the endoscopic diagnosis. For example, as disclosed herein, the dye is released in the colon, including also the right part of the colon.

Thanks to the specific oral solid composition and to the defined administration schedule disclosed herein, the dye orally administered is locally released and also completely absorbed in the intestinal tract, such as in the colon and further such as in the right part of the colon. In that way, that which is disclosed herein avoids any undesired early release or early absorption in anatomical tracts such as the stomach or small intestine not of interest in the endoscopic diagnosis.

The localized absorption of the dye on the intestinal mucosa allows the dye to penetrate in the cells wherein it is retained leading to an improved staining effect, increased contrast and better detection and the related diagnosis.

Improved absorption of the dye is of particular relevance when methylene blue is used as the dye for the endoscopic diagnosis. That follows because methylene blue is a "vital dye" able to be uptaken by the cells in a different way than by the extracellular space. Moreover, oral administration of the composition defined herein according to the administration schedule disclosed herein can lead to detection of a larger number of lesions in the smaller size category, thus improving the endoscopic diagnosis.

The solid compoistion disclosed herein, administered orally as disclosed herein, advantageously can further extensively stain the colonic mucosas, reducing colonoscopy subjectivity due to the endoscopist or operator involved in the endoscopic diagnosis, and consequently improving efficacy of the diagnostic evaluation itself.

The oral composition disclosed herein also can reduce the time involved in the endoscopic diagnosis by avoiding the dead times involved with spraying the dye and then washing it out from the mucosas to be examined.

The examples below also clarify the oral composition and administration schedule disclosed herein, without entailing any restrictions whatsoever with respect thereto.

### EXAMPLES

### Example 1: controlled-release coated tablet for endoscopy (colon)

| Description | UOM | Amt. per tablet |
|---|---|---|
| ***Components*** | | |
| Carmine indigo | mg | 50.0 |
| Lecithin | mg | 5.0 |
| Stearic acid | mg | 10.0 |
| Mannitol | mg | 100.0 |
| Lactose | mg | 50.0 |
| Hydroxyethyl cellulose | mg | 25.0 |
| Sodium starch glycolate | mg | 6.0 |
| Colloidal hydrated silica | mg | 3.0 |
| Magnesium stearate | mg | 2.0 |

| *Coating* | | |
|---|---|---|
| Methacrylic acid copolymer type A (Eudragit L) | mg | 6.0 |
| Methacrylic acid copolymer type B (Eudragit S) | mg | 6.0 |
| Triethyl citrate | mg | 1.2 |
| talc | mg | 5,8 |
| Titanium dioxide | mg | 3.0 |

The applied process provides for mixing the dye with the lecithin surfactant, stearic acid, mannitol and half of the required amount of magnesium stearate. After compacting the mixture, followed by granulation, then cellulose, sodium starch glycolate, colloidal silica and the remaining magnesium stearate are added and, after further mixing, the final compression is then carried out to obtain 250 mg tablets. The tablet is then coated with a mixture of methacrylic copolymers of type A and B, so as to extend the resistance to dissolution in vitro up to a pH ≥7, characteristic of the ileocecal and colon environment.

### Example 2 : controlled-release release coated tablet for endoscopy (colon)

| Description | UOM | Amt. per tablet |
|---|---|---|
| ***Components*** | | |
| Methylene blue | mg | 50.0 |
| Lecithin | mg | 5.0 |
| Stearic acid | mg | 10.0 |
| Mannitol | mg | 100.0 |
| Dibasic Sodium phosphate | mg | 25.0 |
| Hydroxypropyl methylcellulose | Mg | 35.0 |
| Sodium starch glycolate | mg | 6.0 |
| Colloidal hydrated silica | mg | 2.0 |
| Magnesium stearate | mg | 2.0 |

| ***Coating*** | | |
|---|---|---|
| Methacrylic acid copolymer type A (Eudragit L) | mg | 6.0 |
| Methacrylic acid copolymer type B (Eudragit S) | mg | 6.0 |
| Triethyl citrate | mg | 1.2 |
| talc | mg | 5.8 |
| Titanium dioxide | mg | 3.0 |

The preparation process provides for mixing the dye with lecithin, stearic acid and dibasic sodium phosphate, compaction thereof into wafers followed by dry granulation, mixing with the remaining components of the nucleus and the final compression to the weight of 235 mg/tablet. The coating uses methacrylic derivatives as base and an alcohol solvent to facilitate the application phase.

The tablets thus obtained were subjected to dissolution test in vitro, revealing a good resistance to the acid environment and a progressive transfer of the dye in the neutral environment having a pH at 7.2.

### Example 3: controlled release coated tablet for endoscopy (colon)

| Description | UOM | Amt. per tablet |
|---|---|---|
| ***Components*** | | |
| Methylene blue | mg | 200.0 |
| Lecithin | mg | 5.0 |
| Stearic acid | mg | 14.0 |
| Methylhydroxypropyl cellulose | mg | 180.0 |
| Mannitol | mg | 140.0 |
| Microcrystalline cellulose | mg | 140.0 |
| talc | mg | 10.0 |
| Colloidal hydrated silica | mg | 5.0 |
| Magnesium stearate | mg | 6.0 |

| ***Coating*** | | |
|---|---|---|
| Methacrylic acid copolymer type A (Eudragit L) | mg | 16.0 |
| Methacrylic acid copolymer type B (Eudragit S) | mg | 16.0 |
| Triethyl citrate | mg | 6,4 |
| talc | mg | 15.6 |
| Titanium dioxide | mg | 6.0 |

The composition is obtained through advance mixing and granulation of the dye, the lecithin as amphiphilic component, the stearic acid as a component of the lipophilic matrix, mannitol and part of the magnesium stearate. After screening the granules obtained preliminarily, the remaining components and in particular cellulose, capable of producing the hydrophilic matrix structure, are added. The final pharmaceutical form, obtained by compressing the mixture of powders and granules, and weighing about 720 mg, is subjected to coating with a mixture of copolymers of methacrylic derivatives of type A and B, supported by a plasticiser, i.e., triethyl citrate, by a dye pigment, i.e., titanium dioxide, and by an anti-stick agent, such as talc, using ethyl alcohol as a solvent.

The tablet thus obtained resists dissolution in vitro in buffers with pH < 2 and allows a progressive release of the dye substances in buffers with pH > 7 as here below detailed:
- Dissolution % after 2 hours in pH 1 dissolution medium: 0% (spec ≤10%)
- Dissolution % after 4 hour of pH 7.2 dissolution medium: 27%
- Dissolution % after 8 hour of pH 7.2 dissolution medium: 84% (spec >80%)

The same tablets of this Example 3 have been used for a PK Phase I trial, where 200 and 400 mg single doses have been compared and where the following averaged values of the main PK parameters have been recorded: for the 200 mg dose
- mean t_{lag} ≥ 3 hours
- mean tₘₐₓ (hours) 16.10 ± 4.01
- bioavailability compared to injected dose (F_{abs} %): 139.19 ± 52.0
- mean Cₘₐₓ (ng/ml) 1662.2 ± 501.93
- urine excretion (mean % of the dose) = 39.67 ± 19.19
- mean t_{1/2} (hours) 20.19 ± 4.68,
   whereas for the 400 mg dose the main parameters recorded have been:
- mean t_{lag} ≥ 3 hours
- mean tₘₐₓ (hours) 17.67 ± 3.60
- mean Cₘₐₓ (ng/ml) 1635.67 ± 729.57
- urine excretion (mean % of the dose) = 22.99 ± 14.92
- mean t₁₁₂ (hours) 17.25 ± 7.43

### Example 4: controlled-release coated tablet for endoscope (colon)

| Description | UOM | Amt. per tablet |
|---|---|---|
| ***Tablet*** | | |
| Indigo Carmine | mg | 100.0 |
| Sodium Lauryl sulphate | mg | 3.0 |
| Stearic acid | mg | 12.0 |
| Lactose | mg | 130.0 |
| Microcrystalline cellulose | mg | 80.0 |
| Sodium starch glycolate | mg | 10.0 |
| Colloidal hydrated silica | mg | 12.0 |
| Magnesium stearate | mg | 3.0 |

| ***Coating*** | | |
|---|---|---|
| Methacrylic acid copolymer type A | mg | 10.0 |
| Methacrylic acid copolymer type B | mg | 10.0 |
| Triethylcitrate | mg | 8.0 |
| Talc | mg | 6.0 |
| Titanium dioxide | mg | 3.8 |

The process provides for mixing the components of layer 1 and compression thereof, followed by the compression of a mixture of powders and granules obtained from a previous compaction of some components of the layer 2, precisely the dye, lecithin, stearic acid, the microcrystalline cellulose and Mannitol with half of the magnesium stearate, with the remaining co-formulants.

The tablet, weighing about 250 mg, has two differently coloured distinct layers formulated for differentially releasing the dye both in the gastric sector and in the subsequent intestinal sector.

### Example 5: controlled-release coated tablet for endoscopy (colon)

| Description | UOM | Amt. per tablet |
|---|---|---|
| Methylene blue | mg | 25.0 |
| Lecithin | mg | 3.0 |
| Stearic acid | mg | 10.0 |
| Methylhydroxypropyl cellulose | mg | 90.0 |
| Mannitol | mg | 121.0 |
| Microcrystalline cellulose | mg | 60.0 |
| talc | mg | 3.0 |
| Colloidal hydrated silica | mg | 5.0 |
| Magnesium stearate | mg | 3.0 |

| ***Coating*** | | |
|---|---|---|
| Methacrylic acid copolymer type A (Eudragit L) | mg | 8.0 |
| Methacrylic acid copolymer type B (Eudragit S) | mg | 8.0 |
| Triethyl citrate | mg | 3.2 |
| talc | mg | 7.8 |
| Titanium dioxide | mg | 3.0 |

The composition is obtained through ordered mixing of the dye, the lecithin as amphiphilic component, the stearic acid as component of the lipophilic matrix; then the remaining components were added and in particular the celluloses, capable of producing the hydrophilic matrix structure up to completion of the formula. The final pharmaceutical form, obtained by compressing the mixture of powders and granules, unitary weighing of about 320 mg, is subjected to coating with a mixture of copolymers of methacrylic derivatives of type A and B, supported by a plasticiser, triethyl citrate, by a dye pigment, titanium dioxide, and by an anti-sticking agent, such as talc, using ethyl alcohol or water or mixtures thereof as solvent.

The tablet thus obtained revealed in vitro a substantial non-dissolution (<10%) at pH 1 for 2 hours and a progressive dissolution in a simulated intestinal medium with pH 7.2 with a release of:
- about 10% after 1 hour (with specification limit ≤30%)
- about 44% after 4 hours and
- more than 90% at the eighth hour (with specification limit ≥80%).

The tablets have been used also to determine in Human volunteers, subjected to a standard bowel cleansing procedure through the administration of a 4-liters, PEG containing bowel preparation solution (commercially known as Selg^{®} Esse 1000), the PK characteristics of 2 doses of Methylene Blue administered as divided doses individually containing 25 mg of the dye.

The same tablets have been used for a PK Phase I trial, where 100 and 200 mg single doses have been compared and where the following averaged values of the main PK parameters have been recorded:
for the 100 mg dose
   - mean t_{lag} ≥ 3 hours
   - mean tₘₐₓ (hours) 12.0 (individual values 9 - 16)
   - mean Cₘₐₓ (ng/ml) 573.60 ± 175.83
   - urine cumulative excretion (mean % of the dose) in 0 -60 hours = 28.02 ± 11.71
   - mean t_{1/2} (hours) 13.87 ± 5.09
whereas for the 200 mg dose the main parameters recorded have been
   - mean t_{lag} ≥ 3 hours
   - mean tₘₐₓ (hours) 16.0 (individual values 10-24)
   - mean Cₘₐₓ (ng/ml) 1149.12 ± 261.95
   - urine cumulative excretion (mean % of the dose) in 0-60 hours = 38.67 ± 15.8
   - mean t_{1/2} (hours) 15.08 ± 5.85

In order to optimize the way to administer the tablets as function of the mucosal staining results, a clinical trial has been carried out with the above described tablets, using as discriminating parameter a scoring system (TSC) originally created and composed of a number between 0 and 20, calculated as sum of each individual staining score ranging 0 to 5 (where 0 is not stained at all, 1 is "traces", i.e. poor dye traces in colonic mucosa, 2 "detectable", i.e. relevant to a staining of at least 25% of the area, 3 is "acceptable", i.e. relevant to a staining of at least 50% of the area, 4 is "good", i.e. relevant to a staining of at least 75% of the area, 5 is "overstained", i.e. relevant to an overstaining not enabling an endoscopist to see the mucosal surface with the due accuracy in the 100% of the area), measured in the 4 segments of the colonic tract and indicated as right or ascending colon, transverse colon, descending colon and sigma-rectum; this scoring system was used to select the most reliable administration schedule of the dye with the aim of optimizing the tablets administration and the lesions detection possibilities during the colonoscopy procedure.

So, using the tablets formulated as described, the administration schedules has been changed on small groups of patients and the corresponding staining score has been determined. Since the importance of the colonic mucosal staining is that a well stained aspect should be extended to all the colonic segments, not only focused in a single colonic district, an additional parameter has been taken into account: the NSA or Number of Stained Area with staining score ≥2. With the application of these two parameters (TSC and NSA) the determination of the tablets administration schedule in order to obtain the best conditions for the endoscopist to enhance the detection of all the lesions in the colonic mucosa, has been carried out.

In the table below the different administration schedules of the two doses tested are reported with the corresponding measured staining score:
A) for 150 mg dose,
   - with the administration schedule A including 2 tablets (tbs.) before drinking the bowel prep, 2 tbs. after the first litre (L), 2 tbs. after the second L and the mean staining score was 6.8± 4.0 and the mean stained colonic segments (NSA) was 1.3.
   - with the administration schedule B including 6 tablets (tbs.) before drinking the bowel prep, the mean staining score was 2.3±2.4 and the mean stained colonic segments (NSA) was 0,4
   - with the administration schedule C including 6 tablets (tbs.) at the end of the bowel prep, the mean staining score was 8.1±3. 6 and the mean stained colonic segments (NSA) was 1.5.
B) for 200 mg dose,
   - with the administration schedule D including 4 tablets (tbs.) before drinking the bowel prep, 2 tbs. after the first L, 2 tbs. after the second L and the mean staining score was 7.0± 5.0 and the mean stained colonic segments (NSA) was 1.3.
   - with the administration schedule E including 8 tablets (tbs.) at the end of bowel preparation solution the mean staining score was 9.8± 4.4 and the mean stained colonic segments (NSA) was 2.3 .
   - with the administration schedule F including 2 tablets (tbs.) before drinking the bowel prep, 2 tbs. after the first L, 2 tbs. after the second L and 2 tbs. at the end of bowel preparation the mean staining score was 9.3± 4.1 and the mean stained colonic segments (NSA) was 2.2.
   - with the administration schedule G including 2 tablets (tbs.) before drinking the bowel prep, 2 tbs. after the first L, 2 tbs. after the second L and 2 tbs. at the end of bowel preparation the mean staining score (TSC) was 10.5± 7.8 and the mean stained colonic segments (NSA) was 1.5.
   - with the administration schedule H including 4 tbs. after the third L, and 4 tbs. at the end of bowel preparation the mean staining score (TSC) was 10.0 ±3.2 and the mean stained colonic segments (NSA) was 2.1 .
   - with the administration schedule I including 4 tbs. after the second L and 4 tbs. after the third L of bowel preparation the mean staining score (TSC) was 11.4± 3.8 and the meanstained colonic segments (NSA) was 2.8.
   - with the administration schedule J including 2 tablets (tbs.) after the second L 3 tbs: after the third L and 3 tbs. at the end of bowel preparation the mean staining score (TSC) was 11.6± 3.5 and the stained colonic segments (NSA) was 2.6.

Using the same tablets described in Example 5, with a total dose of 200 mg of Methylene blue and an administration schedule of 2 tbs. after the second L, 3 after the third L and 3 at the end of bowel preparation, two Phase II clinical trials have been carried out: A) on 96 completed patients for cancer screening and surveillance, and B) an additional 52 patients belonging to a high risk population, i.e. the patients with long standing Ulcerative Colitis.
A) The cancer screening and surveillance trial had the aim of evaluating the polyp and adenoma detection rate in patients undergoing a full colonoscopy after colonic mucosal staining obtained with Methylene Blue MMX^{®} tablets. Therefore, the primary end-point was to evaluate the polyp detection rate and the adenoma detection rate after colonic mucosal staining

Other Secondary end-point(s) have been set, precisely :
- to classify polyps and adenomas detected after colonic mucosal staining
- to evaluate the serrated lesion detection rate.
- to evaluate the mucosal staining efficacy of Methylene Blue MMX^{®} tablets
- the Bowel cleansing quality was also evaluated according to the validated Boston Bowel Preparation Scale (BBPS).
- to collect data about safety and tolerability of Methylene Blue MMX^{®} tablets after administration of a single dose of 200 mg.

The subjects started the tablets intake in the afternoon before the colonoscopy day and had to drink at least 250 mL of preparation every 15 min, so that the bowel preparation intake could be completed 4 h after.

Measured trial variables:
- frequency of patients with polyps.
- Frequency of patients with adenomas.
- Number of adenomas in the right colon for each patient.
- Number of detected serrated lesions for each patient.
- Mucosal staining score for each area; total staining score.
- Boston bowel preparation score for bowel cleansing preparation quality.
- Time to reach the caecum.
- Time to withdrawal from caecum to exit;
- adverse events,
- vital signs (blood pressure, heart rate, saturation in peripheral blood), body weight. The obtained results are here below summarized.

### 1) Mucosal abnormalities (polyps, adenomas and serrated lesions) in each colonic region per patient (A) and as total number (B)

| Colonic region | Methylene blue MMX® tablets | | | | | |
|---|---|---|---|---|---|---|
| | Number of polyps | | Number of adenomas | | Number of serrated lesions | |
| (A) | | | | | | |
| All regions | 1.8±2.9 | 1.0 (0-20) | 0.9±1.7 | 0 (0-14) | 0.7±1.8 | 0 (0-10) |
| Right colon | 0.6±1.2 | 0 (0-9) | 0.4±1.1 | 0 (0-8) | 0.1±0.4 | 0 (0-2) |
| Caecum | 0.2±0.5 | 0 (0-3) | 0.2±0.4 | 0 (0-3) | 0±0.2 | 0 (0-2) |
| Ascending colon | 0.3±0.6 | 0 (0-3) | 0.2±0.6 | 0 (0-3) | 0.1±0.3 | 0 (0-2) |
| Hepatic flexure | 0.2±0.6 | 0 (0-5) | 0.1±0.5 | 0 (0-4) | 0±0.1 | 0 (0-1) |
| Transverse colon | 0.1±0.4 | 0 (0-2) | 0.1±0.3 | 0 (0-1) | 0±0.2 | 0 (0-1) |
| Splenic flexure | 0.1±0.3 | 0 (0-2) | 0.1±0.3 | 0 (0-2) | 0±0 | 0 (0-0) |
| Descending colon | 0.1±0.3 | 0 (0-1) | 0.1±0.2 | 0 (0-1) | 0±0.2 | 0 (0-1) |
| Sigmoid | 0.4±0.8 | 0 (0-4) | 0.1±0.4 | 0 (0-2) | 0.2±0.6 | 0 (0-3) |
| Rectum | 0.5±1.6 | 0 (0-10) | 0.1±0.6 | 0 (0-5) | 0.4±1,3 | 0 (0-9) |
| (B) | | | | | | |
| All regions | 61 (63.5) | | 45 (46.9) | | 26 (27.1) | |
| Right colon | 32 (33.3) | | 24 (25.0) | | 9 (9.4) | |
| Caecum | 14 (14.6) | | 13 (13.5) | | 2 (2.1) | |
| Ascending colon | 16 (16.7) | | 10 (10.4) | | 5 (5.2) | |
| Hepatic flexure | 9 (9.4) | | 7 (7.3) | | 2 (2.1) | |
| Transverse colon | 12 (12.5) | | 8 (8.3) | | 4 (4.2) | |
| Splenic flexure | 6 (6.3) | | 5 (5.2) | | 0 (0.0) | |
| Descending colon | 7 (7.3) | | 4 (4.2) | | 3 (3.1) | |
| Sigmoid | 21 (21.9) | | 12 (12.5) | | 8 (8.3) | |
| Rectum | 19 (19.8) | | 9 (9.4) | | 12 (12.5) | |

All endoscopic findings were classified by the histopathologist. The detected lesions were predominantly low grade tubular adenomas, hyperplastic serrated lesions, low grade serrated adenomas, low grade tubular-villous adenomas but also high grade adenomas with carcinoma in situ, including tubular-villous, villous and tubular lesions. The mucosa staining efficacy of Methylene Blue MMX® tablets was on average "acceptable" with the 50% of the mucosa stained in all 4 examined colonic regions. Bowel cleansing quality was on average "good" according to the total BBPS score.

### Conclusions:

The polyp detection rate and the adenoma detection rate/patient in the whole colon were on average 1.8±2.9 detected polyps and 0.9±1.7 detected adenomas. The polyp detection rate ranged from 0 to 20 polyps per subject and was higher in the rectum with a maximum of 10 polyps and in the right colon with a maximum of 9 lesions. The adenoma detection rate ranged from 0 to 14 adenomas per subject and was higher in the rectum with a maximum of 5 adenomas. In the right colon, the maximum detection rate was 8 detected adenomas. Serrated lesions ranged from 0 to 10, with the highest prevalence in the rectum with a maximum of 9 lesions.

As summarized in the following table. pPolyps were detected at a frequency of 64%, adenomas at a frequency of 47% and serrated lesions at a frequency of 27.1% (9% of subjects in the right colon, considered at the same severity level than adenomas).

| Number of patients with polyps (%) | Number of patients with adenomas (%) | Number of patients with serrated (%) |
|---|---|---|
| 61 (63.5) | 45 (46.9) | 26(27.1) |

There was a good consistency between the pit pattern scores and histological classification.

The most frequently affected region for polyps was sigmoid and rectum (21.9% and 19.8% respectively) and serrated lesions most frequently in the rectum (12.5%). Considering the 3 areas right, transverse and descending colon, the transverse colon is that with the lowest detection rate, followed by right and descending colon.

The analysis was performed also by subdividing the intraepithelial neoplasiae by size. The rate of detection by lesion size is summarised in the following table. The number of detected polyps, adenomas and serrated lesions < 5mm; mean (±SD) and median (range) are reported.

| Lesion size | Methylene blue MMX® tablets | | | | | |
|---|---|---|---|---|---|---|
| | Number of polyps | | Number of adenomas | | Number of serrated lesions | |
| ≤5 mm | 1.3±2.3 | | 0.5±1.1 | | 0.6±1.7 | |

Smaller lesions (≤5 mm) were predominant in frequency, and that is remarkable inasmuch that the conventional white light colonoscopy, such smaller lesions are the most difficult to detect. Polyps ≤5 mm had a maximum number of 15 detected abnormalities. The maximum number of detected adenomas ≤5 mm was 9 and 10 for the serrated lesions ≤5 mm.

Proportion of subjects with detected polyps by size, with detected adenomas and with detected serrated lesions presented also in the following summary table. The proportion of subjects with detected polyps, adenomas and serrated lesions by colonic region; number (%) of subjects is reported

| Population | Lesion size | Methylene blue MMX® tablets | | |
|---|---|---|---|---|
| | | Subjects with at least one polyp n(%) | Subjects with at least one adenoma n(%) | Subjects with at least one serrated lesion n(%) |
| FAS (N=96) | ≤5 mm | 50(52.1) | 30(31.3) | 23 (24.0) |
| | 6-9 mm | 12 (12.5) | 10(10.4) | 3(3.1) |
| | ≥10 mm | 24 (25.0) | 22 (22.9) | 3(3.1) |

Conclusions:
Efficacy of Methylene Blue MMX^{®} 25 mg modified release tablets was investigated and proved in the detection of the mucosal lesions in all the colonic districts, particularly with the lesions <5mm. A large proportion of patients, compared to data in the literature, has been found affected by the presence of polyps and adenomas, particularly in the sigmoid-rectum district and also in the right colon.

### B) The efficacy of Methylene Blue MMX® 25 mg modified release tablets was investigated in patients with ulcerative colitis with a diagnosis of ≥8 years and colitis activity index<8, This population was chosen because patients with long standing ulcerative colitis have a significantly higher risk for the development of colitis associated colorectal cancers.

The intraepithelial neoplasia detection rate was 16% (8 out of 50 subjects belonging to PP population) with a total of 10 intraepithelial neoplasiae detected in the 8 subjects. Intraepithelial neoplasiae were most frequently found in the rectum-sigma segment (RES), followed by descending colon (DC) and tansverse colon (TC) at the same frequency, and finally by the ascending colon (AC). The number of intraepithelial neoplasiae/subject was 0.2 ± 0.5.

As summarized below, false positive findings represented 8% (4 out of 50 subjects), whilst the false negative findings were 6% (3 out of 50). The method had a sensitivity greater than 50% (precisely 57.1%) and a specificity greater that 90% ( precisely 90.7%.)

Study results are consistent with the higher range of the literature data obtained with the chromo-endoscopy technology a spray of the dye instead of the oral administration of the dye during bowel preparation as disclosed herein. The dye spray technology was able to dramatically reduce the time of examination compared to the random biopsies: in the cited spray chromo-endoscopy trial, intraepithelial neoplasiae were detected at a rate of 15.48% in the same population, with a solution of 0.1% methylene blue sprayed using a catheter.

Detection rate of intraepithelial neoplasiae and true and false positive and negative findings analysis population (N=52).

| Proportion of subjects with intraepithelial neoplasiae | True positive findings | False positive findings | True negative findings | False negative findings |
|---|---|---|---|---|
| 8(15.4) | 4(7.7) | 4 7.7) | 41 (78.8) | 3 (5.8) |

The mucosal staining efficacy of Methylene Blue MMX® tablets was confirmed on average "acceptable" with 50% of the mucosa stained in all 4 examined colonic regions, with the best stained colonic segment resulting in the ascending colon, the region where it's more difficult to find the dysplastic lesions. The majority of subjects had NSA in all 4 regions. Bowel cleansing quality was on average "good" according to the total BBPS score.

Two images of colon endoscopy are below reported to also better clarify the invention. Image 1 shows the contrast enhancing efficacy of the dye according to the present invention in perceiving the deep mucosal tissue structure, with the foci of the glands well defined and darkened in a pre-polyp alteration of the colonic mucosa.

Image 2 shows the semi-continuous blue line defines exactly the borders of the colonic flat lesion that the endoscopist has to take out, allowing a better resolution of the lesion intervention and extraction. The tissue definition is absolutely enhanced owing to the orally administered dye as disclosed herein. With the conventional spraying techniques, the same performance cannot be obtained since little time is available between spray and observation (seconds or a couple of minutes).

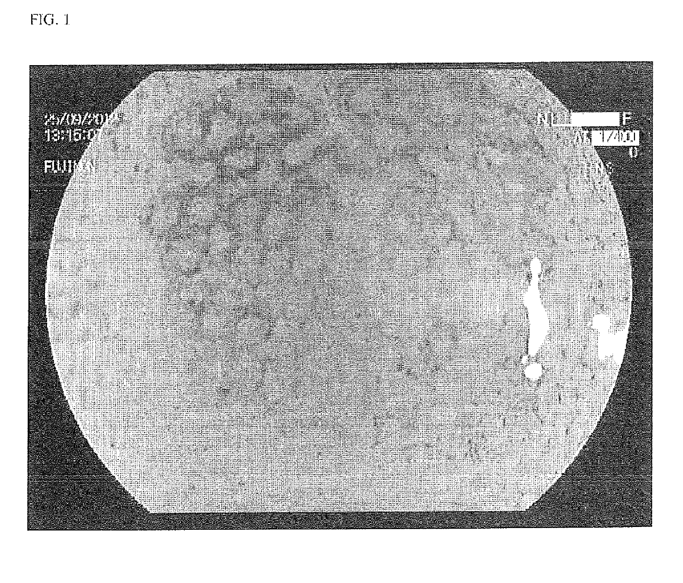

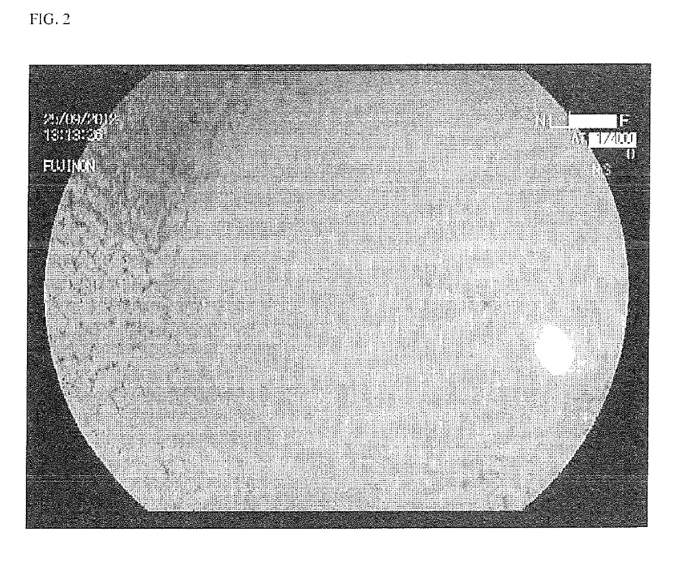

## Claims

1. Solid composition containing at least one dye in association with at least one physiologically acceptable excipient which comprises:
a) a matrix which comprises lipophilic compounds with melting point below 90°C, and optionally amphiphilic compounds, in which said at least one dye is at least partly incorporated,
b) an outer matrix which comprises hydrophilic compounds in which the lipophilic matrix, and optionally the amphiphilic matrix are dispersed;
c) optionally other physiologically acceptable excipients;
d) optional gastro-resistant coating
for use in endoscopic diagnosis, **characterized in that** two or more unit dosages thereof are orally administered to a human according to a fractionated schedule in which a total amount from 50 to 500 mg, preferentially 100 to 400 mg, of said at least one dye is administered to said human in the 48 hours prior to the endoscopic diagnosis.

2. The solid composition according to claim 1, wherein the total dose is fractionated in two or more dosage units containing the same amount of dye, considered the active ingredient.

3. The solid composition according to claim 1, wherein the dosage units are four, six, eight or multiple unit dosages.

4. The solid composition for use according to claim 1, wherein said at least one dye is selected from among methylene blue, congo red, indigo carmine, toluidine blue or mixtures thereof.

5. The solid composition for use according to claim 4, wherein said at least one dye is methylene blue.

6. The solid composition for use according to claim 4, wherein said methylene blue is methylene blue anhydrous or hydrate, preferentially tryhydrate or pentahydrate.

7. The solid composition for use according to claim 1, wherein a total amount from 50 to 500 mg. preferentially from 100 to 400 mg, more preferentially 100 to 250 mg, still more preferentially of 200 mg of said at least one dye is administered to said human.

8. The solid composition for use according to claim 1, wherein a total amount of about 100 mg or about 200 mg of said at least one dye is administered to said human in a single dose administration.

9. The solid composition for use according to claim 2, wherein said unit dosage contains individually from 20 to 100 mg by weight of said at least one dye.

10. The solid composition for use according to claim 2, wherein said unit dosage contains about 25 mg or 50 mg by weight of said at least one dye.

11. The solid composition for use according to claim 2, wherein said unit dosage contains about 200 mg by weight of said at least one dye.

12. The solid composition for use according to claim 1, wherein eight unit dosages thereof each containing about 25 mg by weight of said at least one dye are administered to said human in the 48 hours prior to the endoscopic diagnosis.

13. The solid composition for use according to claim 1, wherein four unit dosages thereof each containing about 50 mg by weight of said at least one dye are administered to said human in the 48 hours prior to the endoscopic diagnosis.

14. The solid composition for use according to claim 1, wherein six unit dosages thereof each containing about 25 mg by weight of said at least one dye are administered to said human in the 48 hours prior to the endoscopic diagnosis.

15. The solid composition for use according to claim 1, wherein four unit dosages thereof each containing about 25 mg by weight of said at least one dye are administered to said human in the 48 hours prior to the endoscopic diagnosis.

16. The solid composition for use according to claim 1, wherein the dosage units thereof are formulated as tablets, preferentially coated tablets, more preferentially gastro-protected coated tablets.

17. The solid composition for use according to claim 1, wherein the dosage units thereof each containing about 25 mg by weight of said at least one dye are administered to said human in the 48 hours prior to the endoscopic diagnosis in a fractionated order at the beginning and/or during and/or at the end of the bowel cleansing preparation administration.

18. The solid composition for use according to claim 17, where the bowel cleansing preparation is consisting of a volume of 2 or more liters of a PEG-based salts containing solution or laxatives-based solution, or laxatives-based solution.

19. The solid composition for use according to claim 17, where the tablets are self administered to the patient by the patient himself, in a well precise order at the beginning, during and at the end of the drinking of the bowel cleansing preparation.

20. The solid composition for use according to claim 17, where the tablets are self administered to the patient by the patient himself, in a well precise order during the second part of administration of the bowel cleansing solution, preferentially taking a tablet each 250 ml of solution during the drinking of the second, third and fourth liter of the bowel cleansing preparation, and more preferentially taking 5 tablets during the second and third liter and 3 tablets during and at the end of the fourth liter of bowel cleansing solution.

21. The solid composition for use according to claim 19, where the bowel cleansing preparation procedure is split in two steps of bowel preparation, preferentially to be administered in two days before the colonoscopy, preferentially part the day before the colonoscopy and part the same day of the colonoscopy and the tablets containing the dye are administered to the patient only during and/or at the end of the bowel preparation step carried out the day before the colonoscopy.

22. The solid composition for use according to claim 19, to be administered fractionated in both the day of the split bowel cleansing preparation, preferentially 6 during the bowel preparation of the day before the colonoscopy and 2 before and/or during and/or at the end of the drinking of bowel cleansing preparation the same day of the colonoscopy.

23. The solid composition for use according to claim 17, wherein eight unit dosages of the composition according to the invention each containing about 25 mg by weight of said at least one dye are orally administered to a human according to a fractionated schedule in which a total amount of about 200 mg of said at least one dye is administered to said human in the 48 hours prior to the endoscopic diagnosis in:
- 0 solid oral compositions after intake of the 1^{st} litre of cleansing solution;
- 2 solid oral compositions after intake of the 2^{nd} litre of cleansing solution
- 3 solid oral compositions after intake of the 3^{nd} litre of cleansing solution; and
- 3 solid oral compositions after intake of the 4^{nd} (and last) litre of cleansing solution.

24. The solid composition for use according to claim 1, wherein eight unit dosages of the composition according to the invention each containing about 25 mg by weight of said at least one dye are orally administered to a human according to a fractionated schedule in which a total amount of about 200 mg of said at least one dye is administered to said human in the 48 hours prior to the endoscopic diagnosis in:
- 0 solid oral compositions after intake of the 1^{st} litre of cleansing solution;
- 4 solid oral compositions after intake of the 2^{nd} litre of cleansing solution
- 4 solid oral compositions after intake of the 3^{nd} litre of cleansing solution; and
- 0 solid oral compositions after intake of the 4^{nd} (and last) litre of cleansing solution.

25. The solid composition for use according to claim 1, wherein the cleansing solution is a saline and/or polyglycol solution, preferably an aqueous solution of polyethylene glycol.

26. The solid composition for use according to anyone of the preceding claims, wherein said at least one dye is administered to said human in the 24 hours prior to the endoscopic diagnosis.

27. The solid composition according to any one of the preceding claims, wherein said unit dosage is a tablet.

28. The solid composition for use according to anyone of the preceding claims, for the endoscopic diagnosis of inflammatory, ulcerative, pre-neoplastic, dysplastic and/or neoplastic pathologies and/or lesions of the gastrointestinal tract, preferably of the colon.

29. The solid composition of claim 28, used to enhance the intestinal mucosal lesion detection the diagnosis of cancerous forms, precancerous forms, interval cancers, adenomas, carcinomas, serrated lesions, intraepithelial neoplasias, dysplasias, polyps, pseudopolyps, pre-polyps or different inflammatory pathologies and/or lesions sessile, flat, peduncolated shape.

30. The solid composition of claim 28, used for the diagnosis of right colon adenomas, right colon polyps and/or interval cancers.

31. The solid composition of claim 28, used for the diagnosis of small size lesions.

32. The solid composition of claim 31, wherein said small size lesions are lesions having a size equal to or less than 5 mm.

33. The solid composition of claim 31, wherein said small lesions are selected among polyps, adenomas and serrated lesions.

34. The solid composition of claim 19, used for enhancing the detection of intestinal mucosal lesions for the early diagnosis in a human previously suffering of other inflammatory pathology as, for example, Inflammatory Bowel Disease (IBD), Ulcerative Colitis or Crohn's Disease.

35. The solid composition of claim 19, used for enhancing the detection of intestinal mucosal lesions of the right part of the colon.

36. The solid composition of claim 1, able to generate after oral administration to human subjects a pharmacokinetic profile with mean t_{lag} ≥ 3 hours.

37. The solid composition of claim 1, able to generate after oral administration to a human a pharmacokinetic profile with mean tₘₐₓ of 16.0 ± 6 hours.

38. The solid composition of claim 1, able to generate after oral administration to a human a pharmacokinetic profile with mean Cₘₐₓ 1149.12 ± 261.95 ng/ml.

39. The solid composition of claim 1, able to generate after oral administration to a human a pharmacokinetic profile with mean urine cumulative excretion in 60 hours of 38.67 ± 15.8 % of the dose.

40. The solid composition of claim 1, able to generate after oral administration to a human a pharmacokinetic profile with mean t_{1/2} 15.08 ± 5.85 hours.

41. The solid composition of claim 1, able to generate after oral administration to a human an Intraepithelial neoplasiae detection outcome with a specificity higher than 80%.

42. The solid composition of claim 1, able to generate after oral administration to a human an Intraepithelial neoplasiae detection outcome with a sensitivity higher than 50%.
